# EUROPÄISCHE PATENTSCHRIFT

(11) **EP 0 399 330 B1**
(45) Veröffentlichungstag und Bekanntmachung des Hinweises auf die Patenterteilung: **28.12.1994**
(21) Anmeldenummer: 90109092.8
(22) Anmeldetag: 15.05.1990
(51) Int. Cl.: C07H 21/00, C07H 21/04, C07H 19/10, C07H 19/20, C07F 9/48, C07F 9/50, C12Q 1/68, C12P 19/34

(54) **Modifiziertes Phosphoramidit-Verfahren zur Herstellung von modifizierten Nukleinsäuren**
Modified phosphoramidite process for preparing modified nucleic acids
Procédé amide-ester phosphoreux modifié pour la préparation d'acides nucléiques modifiés

(30) Priorität: 24.05.1989 DE 3916871
(43) Veröffentlichungstag der Anmeldung: 28.11.1990
(73) Patentinhaber: BOEHRINGER MANNHEIM GMBH, 68298 Mannheim (DE)
(72) Erfinder: Seliger, Heinz Hartmut, Prof. Dr., D-7915 Elchingen-Thalfingen (DE); Berner, Sibylle, Dr. rer. nat., D-8900 Augsburg (DE); Mühlegger, Klaus, D-8121 Polling (DE); von der Eltz, Herbert, Dr. rer. nat., D-8120 Weilheim (DE); Batz, Hans-Georg, Dr. rer. nat., D-8132 Tutzing (DE)

(56) Entgegenhaltungen:
- EP-A- 0 097 373
- EP-A- 0 173 251
- EP-A- 0 216 357
- EP-A- 0 251 283
- EP-A- 0 278 220
- EP-A- 0 285 058
- EP-B- 0 136 543
- WO-A-86/07363
- DD-A- 273 445
- US-A- 4 816 569
- M.J. GAIT: "Oligonucleotide synthesis; a practical approach" IRL PRESS, OXFORD,1984 Seiten 34-49
- BIOCHEMISTRY, Band 27, Nr. 16, August 9, 1988 J GER et al. "Oligonucleotide N-Alkylphosphoramidates: Synthesis and Binding to Polynucleotides" Seiten 7237-7246
- BIOCHIMIE, 67, Nr. 12, 1985 N.TH. THUONG et al. "Chemi- cal synthesis ofnatural and modified oligodeoxynucleo- tides" Seiten 673-684
- CHEMISTRY LETTERS, Nr. 7, 1986 The Chemical Society of Japan, SH. HAMAMOTO etal. "New Approach to the Synthe- sis of Deoxyribonucleoside PhosphoramiditeDerivatives" Seiten 1401-1404
- N.D.SINHA et al. Nucl.Acid Res. 16/6, 2659 (1988)
- WO89/12110

## Beschreibung

Gegenstand der vorliegenden Erfindung sind ein modifiziertes Phosphoramidit-Verfahren zur Herstellung von modifizierten Nukleinsäuren und neue Verbindungen, die in diesem Verfahren eingesetzt werden.

Nukleinsäuren sind eine der Gruppe von Verbindungen, die für das Leben auf der Welt von grundlegender Bedeutung und daher in jedem Lebewesen vorhanden sind. In ihnen ist die genetische Information gespeichert. Sie stellen außerdem ein Kriterium zur Unterscheidung und zum Nachweis verschiedener Arten von Lebewesen dar, da die Nukleinsäuresequenzen für jedes Lebewesen charakteristisch sind. Es hat daher nicht an Versuchen gefehlt, Nukleinsäuren sowohl zu synthetisieren als auch nachzuweisen.

Nukleinsäuren können chemisch oder enzymatisch synthetisiert werden. Die chemische Synthese der natürlich vorkommenden β-konfiguierten Nukleinsäuren hat in jüngster Zeit immer größere Bedeutung erlangt, da so große Mengen von Nukleinsäuren mit einer definierten Nukleotidsequenz hergestellt werden können. Die chemische Synthese hat sich insbesondere für die Synthese von β-konfigurierten Oligonukleotiden etablieren können. Je nach Art der eingesetzten Nukleotidbausteine und der Reaktionsschritte zur Ankrüpfung an das in der Sequenz benachbarte Nukleotid unterscheidet man verschiedene Verfahren:
Im Phosphodiesterverfahren wird ein Nukleosidmonophosphat, in dem alle reaktiven Gruppen mit Ausnahme des Phosphatrests geschützt sind, zusammen mit einem Kopplungsmittel, beispielsweise einem Trialkylarylsulfonsäurechlorid, mit einem weiteren Nukleosid umgesetzt, in dem alle reaktiven Reste bis auf die Hydroxygruppe, an der die Reaktion stattfinden soll, geschützt sind. Die Ausbeuten in diesem Verfahren sind gering, vor allem deswegen, weil bei den Kondensationsschritten zum Aufbau der Oligonucleotidkette unerwünschte Nebenreaktionen an den nichtveresterten OH-Funktionen der Internucleotid(phosphat)brücke ablaufen und zu komplexen Reaktionsgemischen führen. Es weist ferner den großen Nachteil auf, daß die entstandenen Phosphorsäurediester nur in wenigen protischen Lösungsmitteln, in denen die Veresterung durchgeführt werden muß, löslich sind. Solche Lösungsmittel wie Pyridin, Dimethylformamid oder Dimethylsulfoxid haben bekannte Nachteile, wie z.B. hohe Siedepunkte. Aufgrund des polaren Charakters der Phosphodiester-Derivate muß die Isolierung und Aufreinigung über Ionenaustauscher erfolgen und kann nicht in einfacher Weise z.B. über Kieselgel unter Verwendung niedrig siedender Lösemittel (wie z.B. Dichlormethan) erfolgen.

Den Nachteil der Unlöslichkeit der Produkte in zahlreichen organischen Lösungsmitteln umgeht die Phosphotriestermethode.

Die Phosphotriestermethode arbeitet mit einem Phosphorsäurederivat, das nur 1 reaktive Gruppe, aber 2 durch unterschiedliche Schutzgruppen geschützte Hydroxygruppen direkt am Phosphoratom aufweist. Nach der Umsetzung mit dem ersten Nukleosid wird eine der Schutzgruppen abgespalten und die entstandene Hydroxygruppe kann dann für die Reaktion mit dem zweiten Nukleosid aktiviert werden. Diese Vorgehensweise bedeutet, daß es erforderlich ist, zwei zusätzliche Reaktionsschritte an dem Nukleosidphosphat auszuführen, was zu einer Ausbeuteverringerung an aktiviertem Nukleosidphosphat führt.

Eine besonders vorteilhafte Methode, die mit weniger Reaktionsschritten an den relativ teuren Synthesebausteinen auskommt, ist als Phosphoramiditverfahren bekannt geworden. (Gait, M.J. et al., Oligonucleotide Synthesis: A Practical Approach, IRL Press Oxford). Hier werden keine Phosphorsäurederivate, sondern Derivate der phosphorigen Säure, sogenannte Phosphoramidite eingesetzt. Folgende Reste sind an dem trivalenten Phosphoratom angebracht:
- eine reaktive Gruppe, beispielsweise ein Halogenatom, das die Verknüpfung mit dem ersten Nukleosid ermöglicht,
- eine sekundäre Aminogruppe, mit der nach Aktivierung die Verknüpfung mit dem zweiten Nukleosid bewirkt werden kann, und
- eine durch eine Schutzgruppe maskierte Hydroxygruppe.

Im ersten Schritt des Phosphoramiditverfahrens wird das Derivat der phosphorigen Säure mit einem ersten Nukleosid umgesetzt; dabei ersetzt das Nukleosid die reaktive Gruppe. Im zweiten Schritt wird selektiv der Ersatz der sekundären Aminogruppe durch ein zweites Nukleosid bewerkstelligt. Als Aktivierungs-Reagens findet im zweiten Schritt meist ein Tetrazol Verwendung. In einem folgenden Schritt wird die Nukleotidsequenz oxidiert, beispielsweise mit Jod, und die Schutzgruppe abgespalten. Das Phosphoramiditverfahren ist in einer Variante als Festphasenverfahren beschrieben. Dabei ist die wachsende Nukleotidsequenz an eine Festphase gebunden. Die Abtrennung von überschüssigen Synthesereagentien und -bausteinen sowie die Reinigung der Oligonukleotidsequenz ist dadurch stark vereinfacht worden. Kommerziell erhältliche Nukleinsäuresyntheseautomaten arbeiten nach diesem Verfahren. Sie sind in ihrer Konstruktion z.B. auf die spezifischen Schritte des Phosphoramiditverfahrens abgestimmt.

Nukleinsäuren mit bekannter Nukleotidsequenz finden besonders Anwendung zum spezifischen Nachweis von DNA in biologischem Probenmaterial.

In solchen Nachweisverfahren wird die Eigenschaft ausgenutzt, daß die einzelnen Stränge von Nukleinsäuren mit anderen einzelsträngigen Nukleinsäuren unter Bildung eines Doppelstranges reagieren können, wenn die Einzelstränge zueinander komplementäre Nukleotidsequenzen aufweisen und beide dieselbe Konfiguration an C-1 der Ribose (α bzw. β) haben. Da die natürlich vorkommenen Nukleinsäuren hinsichtlich der Verknüpfung von Basen und Zuckern β-konfiguriert sind, kommen als komplementäre Nukleinsäuren insbesondere die β-Nukleinsäuren in Frage. Der Vorgang der Doppelstrangbildung wird Hybridisierung genannt.

Die Bildung eines Doppelstrangs kann nachgewiesen werden, wenn zur Hybridisierung mit der einzelsträngigen Nukleinsäure eine modifizierte einzelsträngige komplementäre Nukleinsäure eingesetzt wird. Anschließend wird die Menge der hybridisierten Nukleinsäuren über die Modifizierung, die beispielsweise eine radioaktive Markierung sein kann, bestimmt.

Zur Synthese von modifizierten Nukleinsäuren kann entweder eine schon vorhandene natürliche Nukleinsäure chemisch oder enzymatisch modifiziert werden, oder die Nukleotidsequenz kann unter Zuhilfenahme bereits modifizierter Nukleotid-Bausteine synthetisiert werden.

Durch Modifikation bereits fertig synthetisierter Nukleinsäuren an den Enden, wie sie beispielsweise für das 5′-Ende in der WO 86/07363 vorgeschlagen wird, können jedoch nur Nukleinsäuren hergestellt werden, die ein einziges modifiziertes Nukleotid pro Einzelstrang beinhalten. Methoden zur Bestimmung der Menge an Nukleinsäuren mit dieserart modifizierten Nukleinsäuren als Sonden sind daher wenig empfindlich.

Daher wurde beispielsweise in der EP-A-0 173 251 vorgeschlagen, die Basen Von kompletten Nukleinsäuren durch chemische Reaktionen zu modifizieren. Dazu sind jedoch mehrere Reaktionsschritte an der Nukleinsäure erforderlich und die Modifikationsrate ist davon abhängig, ob die Nukleinsäure Basen mit freien Aminogruppen enthält, deren Modifizierung die Fähigkeit der Hybridisierung mit komplementären Nukleinsäure nicht beeinträchtigt.

In Jäger et al. (Biochemistry Vol. 27, S. 7237 (1988)) wird die Herstellung eines Dinukleotids beschrieben, welches eine Modifikation am Phosphoratom trägt. Die Modifikation besteht in einer über einen Linker gebundenen primären Aminogruppe und wird in einem dem herkömmlichen Phosphoramiditverfahren ähnlichen Verfahren eingeführt.

Dieses Verfahren kann jedoch nicht auf den herkömmlichen Syntheseautomaten der Phosphoramiditmethode ausgeführt werden. Ein weiterer Nachteil ist, daß keine zusätzlichen Nükleotide mehr angefügt werden können, da die freie Aminogruppe mit den dazu benutzten elektrophilen Reagenzien selbst reagiert.

In US-A-4,816,569 ist ein Verfahren zur Herstellung von Nükleotidsequenzen nach dem Prinzip der Phosphortriestermethode beschrieben, bei dem an ein am 3'-Phosphatrest modifiziertes Mononucleotid weitere Mononucleotideinheiten angehängt werden, von denen die letzte am 5'-Ende modifiziert sein kann. Eine auf diesem Wege erhältliche Mehrfachmarkierung ist relativ umständlich, da mindestens zwei verschieden modifizierten Mononukleotideinheiten benötigt werden.

In EP-A-0 285 058 ist ein Verfahren zum Einbau von basenmodifizierten Mononucleotiden in Nükleinsäuren beschrieben und sind Mono- und Polynucleotide beansprucht, an die eine Reporter-Gruppe über ein Phosphoratom gebunden ist.

Jedes der im Stand der Technik vorhandene Verfahren weist daher beträchtliche Nachteile auf.

Aufgabe der vorliegenden Erfindung war es, die Nachteile der bekannten Verfahren zu vermeiden und insbesondere ein mit einfachen Ausgangsstoffen in wenigen Reaktionsschritten unter hohen Ausbeuten ausführbares Verfahren zur Synthese am Phosphatrest modifizierter β-konfigurierter Nukleinsäuren an Festphasen zur Verfügung zu stellen.

Ein Gegenstand der Erfindung ist ein Verfahren zur Herstellung von Nukleotidsequenzen der Formel IX 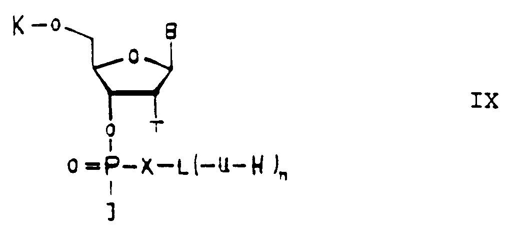
in der
- K: Wasserstoff oder das Phosphoratom des Phosphatrests eines weiteren Nukleotids oder einer Nukleotidsequenz
- J: eine Hydroxygruppe oder ein 5′-Sauerstoffatom eines weiteren Nukleotids oder einer Nukleotidsequenz
- B: eine natürliche oder modifizierte Nukleobase
- T: Wasserstoff, Niederalkyl, Azid, Niederalkyloxy oder eine Hydroxygruppe bedeuten,
- X: Sauerstoff oder Schwefel,
- L: ein (n + 1)-valentes Brückenglied,
- U: Sauerstoff, Schwefel, Stickstoff oder N-H und
- n: eine natürliche Zahl von 1 bis 200 bedeuten,
durch Reaktion eines Nukleosidphosphoramidits mit einem weiteren Nukleotid, das eine freie Hydroxylgruppe aufweist, und Oxidation der entstandenen Nukleotidsequenz zu einem Phosphat, dadurch gekennzeichnet, daß als Nukleosidphosphoramidit eine Verbindung der Formel I 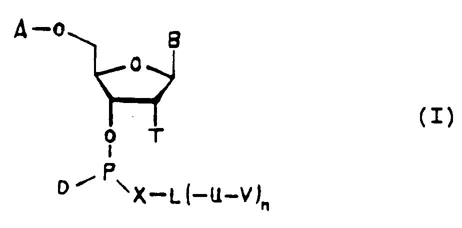
eingesetzt wird, wobei
- A: eine Sauerstoffschutzgruppe, ein Nukleotid oder ein Oligonukleotid,
- B: eine natürliche oder modifizierte Nukleobase,
- X: Sauerstoff oder Schwefel,
- L: ein (n + 1)-valentes Brückenglied,
- T: Wasserstoff, Niederalkyl, N₃, Niederalkoxy oder eine gegebenenfalls geschützte Hydroxygruppe,
- U: Sauerstoff, Schwefel, Stickstoff oder N-H,
- V: eine abspaltbare Schutzgruppe
- n: eine natürliche Zahl von 1 bis 200 und
- D: ein sekundärer Aminrest bedeuten.

Verfahren zur Herstellung von Nukleinsäuren über das sogenannte Phosphoramiditverfahren sind prinzipiell bekannt, beispielsweise aus Biochimie 1985, 67, 673-684. Das Verfahren der vorliegenden Erfindung unterscheidet sich insbesondere dadurch von den Verfahren des Standes der Technik, daß ein anderes Nukleosidphosphoramidit, nämlich das der Formel I, als Ausgangsstoff eingesetzt wird.

Bevorzugter Rest A der Formel I ist eine Sauerstoffschutzgruppe. Schutzgruppen, die für den Schutz der 5′-Hydroxygruppe in Nukleotidsynthesen geeignet sind, sind bekannt. Besonders oft verwendet werden sauer abspaltbare Schutzgruppen, wie die Triphenylmethylgruppe oder die Dimethoxytriphenylmethylgruppe.

Wenn der Rest A ein Nukleotid oder Oligonukleotid bedeutet, so kann es sich um ein natürliches oder ein modifiziertes Nukleotid bzw. Oligonukleotid handeln. Die Nukleotide sind gegenüber den Oligonukleotiden bevorzugt, da der Syntheseaufwand bei Oligonukleotiden erhöht ist. Bei den Nukleotiden bzw. Oligonukleotiden des Rests A kann es sich auch um erfindungsgemäß hergestellte Reste halten. Reaktive Gruppen der Nukleotide bzw. Oligonukleotide des Rests A sind bevorzugt durch geeignete Schutzgruppen geschützt. Insbesondere ist die endständige 5′-Hydroxygruppe des Nukleotids bzw. Oligonukleotids des Restes A durch eine Sauerstoffschutzgruppe geschützt. Diese Sauerstoffschutzgruppe hat insbesondere die oben unter Rest A genannte Bedeutung.

Die natürliche Nukleobase des Rests B ist bevorzugt Adenin, Thymin, Cytosin, Uracil oder Guanin. Bei den modifizierten Basen kann es sich beispielsweise um im Ring oder in den Substituenten in der Struktur veränderte Basen handeln. Beispiele sind 7-Deazaguanin oder 5-Aminoalkyluracil oder 8-Aminohexyl-amino-adenin. Bevorzugt sind solche Basen, bei denen die Watson-Crick Basenpaarung mit einer komplementären Nukleinsäure nicht oder sehr wenig beeinflußt wird.

Der Rest T kann die Ribo- oder Arabino-Konfiguration aufweisen. Bevorzugt ist die Ribo-Konfiguration. Als Schutzgruppe des Hydroxyrests kommen insbesondere basisch, sauer oder nucleophil abspaltbare Gruppen, bevorzugt die t-Butyldimethylsilyl oder Triisopropylsilyl-Gruppe in Frage.

Die Schutzgruppe V ist bevorzugt eine selektiv abspaltbare Schutzgruppe. Bevorzugt ist eine Schutzgruppe, die gleichzeitig unter den Bedingungen abgespalten wird, unter denen die fertige Nukleotidsequenz vom festen Träger abgespalten wird. Nicht bevorzugt sind daher sauer abspaltbare Schutzgruppen, etwa der Bedeutung in A. Besonders bevorzugt sind alkalisch oder ammoniakalisch abspaltbare Schutzgruppen; als besonders günstig hat sich die Fluorenylmethoxycarbonylgruppe oder die Trifluoracetylgruppe erwiesen.

Unter Alkylgruppen und Alkoxygruppen werden Reste mit 1 bis 6, bevorzugt 1 bis 4 Kohlenstoffatomen verstanden.

Die Verbindungen der Formel I können aus Verbindungen der Formel II 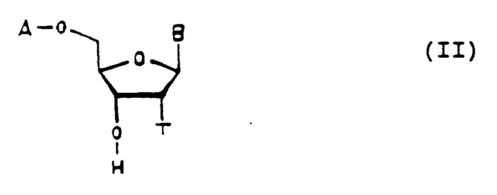
in der
- A: eine Sauerstoffschutzgruppe, ein Nukleotid oder ein Oligonukleotid,
- B: eine natürliche oder modifizierte Nukleobase und
- T: Wasserstoff, eine (gegebenenfalls geschützte) Hydroxygruppe, Niederalkyl, N₃ oder Niederalkyloxy
bedeuten,
durch Reaktion mit Phosphanen der Formel III 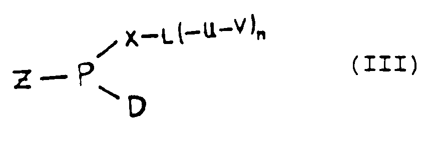
in der
- Z: Halogen,
- X: Sauerstoff oder Schwefel,
- L: ein mindestens bivalentes Brückenglied,
- U: Sauerstoff, Schwefel, Stickstoff oder N-H
- V: eine abspaltbare Schutzgruppe,
- n: eine natürliche Zahl von 1 bis 200 und
- D: ein sekundärer Aminrest
bedeuten,
hergestellt werden. Die Reaktionsbedingungen können vom Fachmann analog zu denen gewählt werden, wie sie für die Nukleosidphosphoramidite des Standes der Technik bereits beschrieben sind. Jedoch muß darauf geachtet werden, daß dabei keine Reagenzien verwendet werden, bei deren Verwendung die Schutzgruppe V abgespalten werden kann. Diese Reaktionsbedingungen sind dem Fachmann für die einzelnen Schutzgruppen bekannt.

Das Phosphan der Formel III kann in einfacher Weise aus kommerziell erhältlichen Ausgangsstoffen synthetisiert werden. Die bevorzugte Reihenfolge der Herstellungsreaktionen sieht zunächst die Reaktion mit einem sekundären Amin vor, da dieses ein billiger Rohstoff ist. Bei diesem Reaktionsschritt können daher notfalls auch Ausbeuteverluste durch unspezifische Reaktion in Kauf genommen werden. Das Phosphan der Formel III wird bevorzugt dadurch hergestellt, daß eine Verbindung der Formel (VI)

P(-Z)₃ (VI)

in der Z eine gut austretende Gruppe bedeutet, mit einem sekundären Amin der Formel (VII)

H-D (VII)

in der
- D: ein sekundärer Aminrest
bedeutet,
umsetzt, und das Produkt mit einer Verbindung der Formel VIII

H-X-L(-U-V)ₙ (VIII)

in der
- X: Sauerstoff oder Schwefel,
- L: ein (n + 1)-valentes Brückenglied,
- U: Sauerstoff, Schwefel, Stickstoff oder N-H,
- V: eine abspaltbare Schutzgruppe,
- n: eine natürliche Zahl von 1 bis 200
bedeuten, reagieren läßt und das entstandene Produkt abtrennt.
Der Rest Z ist Halogen, bevorzugt Chlor.

Verbindungen der Formel VII sind insbesondere dem Fachmann bekannte sekundäre Amine der Formel H-NR¹R² wobei R¹ und R² gleich oder verschieden sind und primäre, sekundäre oder tertiäre Alkylreste mit 1-10 Kohlenstoffatomen sind, oder zusammen einen gegebenenfalls alkylverzweigten Cycloalkylrest mit 5-7 Kohlenstoffatomen, der ein oder zwei Stickstoff-, Sauerstoff- und/oder Schwefelatome als Heteroatome enthalten kann, darstellen, oder N R¹ R² einen Imidazolyl-, Triazolyl-, Tetrazolyl-, 3-Nitro-1,2,4-triazolyl-,Thiazolyl-, Pyrrolyl-, Benztriazolyl- oder Benzhydroxytriazolylrest bedeutet. Als besonders bevorzugte Amine haben sich Diisopropylamin und Morpholin erwiesen.

Als Brückenglied sind insbesondere lineare oder verzweigte, gesättigte oder ungesättigte Kohlenwasserstoffe mit 1 bis 10, bevorzugt 2 bis 6 Kohlenstoffatomen zu nennen. Die Kohlenwasserstoffkette kann durch Heteroatome, beispielsweise Sauerstoff oder Schwefel unterbrochen sein. Das Brückenglied kann auch aliphatische oder aromatische Ringsysteme beinhalten. Das Brückenglied kann auch weitere Heteroatome tragen. Im Hinblick auf die Reaktionen, die mit Verbindungen, die dieses Brückenglied beinhalten, im erfindungsgemäßen Verfahren ausgeführt werden sollen, sind jedoch solche Brückenglieder auszuschließen, die freie unsubstituierte oder primäre Aminogruppen oder Hydroxygruppen als Substituenten aufweisen. Das Brückenglied ist über n-kovalente Bindungen mit n Gruppen U verbunden. Die bevorzugte Anzahl von n beträgt 1 bis 200.

Die Verbindungen der Formel III haben gegenüber den Phosphanen des Standes der Technik den Vorteil, sowohl zur Synthese von Nukleosidphosphoramiditen für die Phosphoramiditsynthese von Nukleinsäuren einsetzbar zu sein, als auch in geschützter Form eine reaktive Gruppe aufzuweisen; diese kann als Verknüpfungsstelle für detektierbare Reste dienen.

Das erfindungsgemaße Verfahren zur Herstellung von Nukleotidsequenzen umfaßt insbesondere folgende Schritte:
- Kopplungsreaktion eines Nukleosidphosphoramidits der Formel I mit einem Nukleosid, das eine freie Hydroxygruppe aufweist. Das Nukleosid mit der freien Hydroxygruppe ist bevorzugt kovalent an einen festen Träger gebunden. Die übrigen reaktiven Gruppen des Nukleosids, wie Aminogruppen, Carbonylgruppen oder weitere Hydroxygruppen sind bevorzugt durch Schutzgruppen geschützt, die unter den Bedingungen der Kopplungsreaktion stabil sind. Bevorzugt ist eine eventuell vorhandene 2′-Hydroxygruppe am Zuckerrest durch eine t-Butyldimethylsilyl-gruppe geschützt. Die freie Hydroxygruppe ist bevorzugt die 5′-Hydroxygruppe des Zuckerrests.
   Das Nukleosid kann ein Mononukleosid, ein Oligo- oder Polynucleotid sein. Bevorzugt ist es jedoch ein Mononucleosid, Oligo- oder Polynukleotid aus 2 bis 200, bevorzugt 20 bis 60 Nukleotidbausteinen. Die Nukleotidbausteine können natürliche oder modifizierte Nukleotide sein.
   Bei dem Nukleosid kann es sich auch um ein in erfindungsgemäßer Weise modifiziertes Nukleosid handeln.
- Anschließend wird die an die Festphase gebundene Nukleotidsequenz oxidiert. Als bevorzugtes Oxidationsmittel hat sich Jod erwiesen.
- Daraufhin wird bevorzugt ein "capping"-Schritt durchgeführt. Dies geschieht nach bekannten Methoden.
- Selektive Abspaltung der Schutzgruppe A bzw. der Sauerstoffschutzgruppe der endständigen 5′-Hydroxygruppe des Nukleotids oder Oligonukleotids des Rests A. Im bevorzugten Fall, wenn die Sauerstoffschutzgruppe des Rests A eine sauer abspaltbare Schutzgruppe wie eine Dimethoxytriphenylmethylgruppe ist, kann sie beispielsweise durch Dichloressigsäure abgespalten werden.
- Diese ersten Schritte können nun, falls gewünscht, wiederholt werden. Als Mononukleosidphosphoramidit kann dabei ein herkömmliches Mononukleosidphosphoramidit oder eines der Formel I eingesetzt werden.
- Sobald die gewunschte Lange der Nukleotidsequenz erreicht ist, werden die Schutzgruppen V abgespalten. Im Falle der Aminoschutzgruppe hat sich der Trifluoracetyl- oder der Fluorenylmethoxycarbonylrest (Fmoc) als besonders vorteilhaft erwiesen.
- Anschließend wird die Nukleotidsequenz in bekannter Weise vom festen Träger abgespalten. Die Bedingungen richten sich nach der Art der kovalenten Bindung und werden nicht durch die erfindungsgemäße Modifizierung beeinflußt.
   Besonders bevorzugt sind jedoch solche Bedingungen, unter denen die Abspaltung der Schutzgruppe V und die Abspaltung der Nukleotidsequenz vom Träger gleichzeitig ablaufen. Dies kann beispielsweise bei Verwendung eines über 3′-0-Succinyl an CPG (controlled pore glass) gebundenen Trägers und der Fmoc-Schutzgruppe als Rest V bewerkstelligt werden, indem Alkali, vorzugsweise konzentrierte wässrige Ammoniaklösung oder Aminlösung als Abspaltungsreagens verwendet wird.
- Meist wird ein Reinigungsschritt, beispielsweise eine Reinigung mittels HPLC chromatographisch oder/und eine Dialyse angeschlössen. Hier gelten die bei der Oligonucleotidsynthese gebräuchlichen Bedingungen.

All diesen Schritten ist gemeinsam, daß außer der Tatsache, daß ein anderes Nukleosidphosphoramidit eingesetzt wird und daß anstelle der Reagenzien zur Abspaltung der Sauerstoffschutzgruppen am Phosphatrest des Standes der Technik Reagenzien zur Abspaltung der Schutzgruppe V eingesetzt werden, keine Änderungen im herkömmlichen Verfahrensablauf vorgenommen werden müssen. Insbesondere ist die Anzahl der Schritte die gleiche oder kleiner wie bei dem herkömmlichen Phosphoramiditverfahren. Daher ist das erfindungsgemäße Verfahren in den erhältlichen Nukleinsäuresynthesizern für die Phosphoramiditsynthese ohne apparative Änderungen durchführbar.

Die so hergestellte Nukleotidsequenz der Formel IX weist bevorzugt 2 bis 200, besonders bevorzugt 20 bis 60 Nukleotidbausteine auf. Davon sind bevorzugt 10 bis 80%, besonders bevorzugt 20 bis 50% der Nukleotidbausteine aus Nukleosidmonophosphaten der Formel I entstandene am P-Atom modifizierte Nukleotidbausteine, wobei pro Nukleotidsequenz mehr als ein Nukleotidbaustein modifiziert ist. Diese modifizierten Nukleotidbausteine weisen in der Sequenz bevorzugt einen Abstand von 2-5 Nukleotiden zueinander auf.
Die Verbindungen der Formel IX sind vielseitig einsetzbar.

Aus den erfindungsgemäß hergestellten Nukleotidsequenzen der Formel IX können auf einfache Weise Nukleotidsequenzen hergestellt werden, die mehrere detektierbare Reste oder Reste aufweisen, die in einen detektierbaren Rest überführt werden können. Es hat sich erwiesen, daß der Nachweis von Nukleinsäuren damit empfindlicher wird.

Ein weiterer Gegenstand der Erfindung ist daher ein Verfahren zur Herstellung einer Nukleotidsequenz der Formel V, 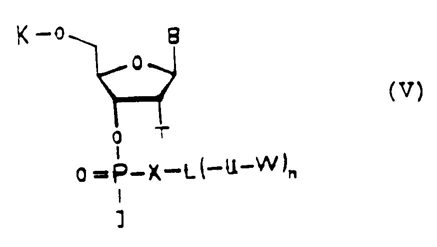
in der
- K: Wasserstoff oder das Phosphoratom des Phosphatrests eines weiteren Nukleotids oder einer Nukleotidsequenz
- J: eine Hydroxygruppe oder ein 5'-Sauerstoffatom eines weiteren Nukleotids oder einer Nukleotidsequenz
- B: eine natürliche oder modifizierte Nukleobase
- T: Wasserstoff, Niederalkyl, Azid, Niederalkyloxy oder eine Hydroxylgruppe bedeuten
- W: ein detektierbarer Rest oder ein Rest ist, der in einen detektierbaren Rest überführt werden kann und X, L, U die oben angegebene Bedeutung haben, und
- n: eine natürliche Zahl von 1 bis 200 ist, wobei mehr als ein Nukleotidbaustein gemäß Formel V am Phosphoratom modifiziert ist;
wobei anschließend an die oben genannten Schritte die entstandene Nukleotidsequenz der Formel IX mit einer Verbindung der Formel IV

Y-W (IV),

umgesetzt wird, wobei
- Y: eine reaktive Gruppe und
- W: ein detektierbarer Rest oder ein Rest ist, der in einen detektierbaren Rest überführt werden kann.

Als reaktive Gruppe Y kommt beispielsweise eine leicht nukleophil substituierbare Gruppe oder eine elektrophile Gruppe in Frage. Verbindungen der Formel IV sind beispielsweise Carbonsäurehalogenide.

Elektrophile Gruppen sind beispielsweise die Gruppen in aktivierten Estern oder Anhydriden. Ein bevorzugter Ester ist beispielsweise der N-Hydroxysuccinimidester von Haptenen, wenn diese eine Carboxylgruppe aufweisen.

Das weitere Nukleotid in der Bedeutung der Reste K bzw. J kann ein natürliches oder ein modifiziertes Nukleotid sein. Die Nukleotidsequenz in der Bedeutung der Reste K bzw. J kann sowohl natürliche, als auch modifizierte Nukleotidbausteine enthalten. Die Nukleotidsequenz der Formel V weist bevorzugt 2 bis 200, besonders bevorzugt 20 bis 60 Nukleotidbausteine auf. Davon sind bevorzugt 10 bis 80%, besonders bevorzugt 20 bis 50% der Nukleotidbausteine aus Nukleosidmonophosphaten der Formel I entstandene Nukleotidbausteine, wobei pro Nukleotidsequenz mehr als ein Nukleotidbaustein modifiziert ist.

Der Rest W kann nieder-, wie auch hochmolekularer Struktur sein. Bevorzugte niedermolekulare Reportermoleküle sind Farbstoffe und Haptene; bevorzugte hochmolekulare Gruppen sind z.B. Enzyme oder immunologisch aktive Substanzen wie Antigene oder Antikörper. Besonders bevorzugt sind Haptene. Von diesen sind insbesondere solche bevorzugt, die unter normalen Bedingungen in Körperflüssigkeiten nicht vorkommen, wie beispielsweise Digoxigenin. Als besonders vorteilhaft haben sich Haptene und insbesondere Digoxigenin als immunologisch aktive Substanz erwiesen, da die sie aufweisenden Nukleotidsequenzen durch die Modifizierung nicht sehr in ihrem Molekulargewicht verändert werden und so als Längenstandards beispielsweise in der Gelchromatographie eingesetzt werden können.

Es hat sich herausgestellt, daß das erfindungsgemäße Verfahren zur Herstellung von Nukleotidsequenzen weiterhin folgende Vorteile gegenüber dem Stand der Technik aufweist:
- Dadurch, daß die Modifizierung am Phosphoratom angebracht ist, wird die Basenpaarung der gebildeten Nukleotidsequenz mit einer komplementären Nukleotidsequenz nicht beeinträchtigt.
- Die gebildeten Nukleotidsequenzen werden von Polymerasen als Primer akzeptiert.
- Die Modifizierung kann zusätzlich zu anderen Modifizierungen, beispielsweise des Zuckerrests oder der Base, oder zu 3′- oder 5′-Endmarkierungen eingeführt werden.
- Es handelt sich um ein Verfahren, das eine konvergente Synthese der benötigten Bausteine beinhaltet. Solche Verfahren sind besonders vorteilhaft, da die Ausbeuten insbesondere an den teuren Nukleotidbausteinen hoch gehalten werden können.
- Es können zur Synthese der Nukleosidphosphoramidite die leicht erhältlichen, natürlich vorkommenden β-Nukleoside eingesetzt werden.
- Bei gleichbleibender oder sogar verringerter Anzahl von Reaktionsschritten wurde es möglich, die bekannten Vorteile des Festphasen-Phosphoramiditverfahrens zur Synthese von Nukleotidsequenzen zur Synthese von am Phosphatrest modifizierten Nukleotidsequenzen zu nutzen.
- Durch das erfindungsgemaße Verfahren ist es möglich, eine ganz spezielle Zahl von Modifikationen an ganz bestimmten Stellen der Sequenz einzuführen.
- Die gebildete modifizierte Nukleotidsequenz ist universell einsetzbar. Beispielsweise können verschiedene detektierbare Reste gewählt werden.
- Dadurch, daß die detektierbaren Reste nicht von Anfang an in den Nukleosidphosphoramiditen vorhanden sind, werden Komplikationen, während der chemischen Synthese des Nucleotides wie sie beispielsweise bei Enzymmarkierungen oder anderen empfindlichen Reportergruppen zu erwarten sind, vermieden.
- Die sterische Hinderung durch Reportermoleküle kann die Ausbeute und Effizienz von Oligonucleotid-Synthesen verringern. Dieser Nachteil wird im erfindungsgemäßen Verfahren vermieden.

Die Nukleotidsequenzen der Formel V können vorteilhaft in Verfahren zum Nachweis von Nukleinsäuren in einer Probe durch Inkontaktbringen der Probe mit einer dazu im wesentlichen komplementären Nukleinsäure, Behandlung des Gemischs unter Bedingungen, die zur Hybridisierung zueinander komplementärer Nukleinsäuren führt, und Nachweis des detektierbaren Restes als zur Proben-DNA komplementäre Nukleotidsequenz eingesetzt werden. Der Nachweis des detektierbaren Restes kann nach bekannten Methoden erfolgen. Wenn der detektierbare Rest eine immunologisch aktive Substanz ist, so kann der Rest mit einem markierten immunologischen Partner umgesetzt werden. Anschließend wird dann die Markierung gemessen. Als Rest W sind im Falle dieser Verwendung der erfindungsgemäßen Nukleinsäuren Haptene, insbesondere Digoxigenin, bevorzugt.

Ebenso sind sie als Primer in der enzymatischen Synthese von doppelsträngigen Nukleinsäuren aus einzelsträngigen Nukleinsäuren geeignet. Die entstehende doppelsträngige Nukleinsäure enthält dann die Nukleotidsequenz in mindestens einem der beiden Stränge.

Die Erfindung wird durch die folgenden Beispiele erläutert:

### Beispiel 1:

### 2-(9-Fluorenylmethoxycarbonyl-)aminoethanol

In einem 1 l-Rundkolben werden 68,0 g (ca. 200 mMol) 9-Fluorenylmethoxycarbonyl-N-hydroxy-succinimidester (Fmoc-O-Su) unter Rühren in 300 ml Dioxan gelöst. Zu der klaren Lösung werden nacheinander 40 g in 200 ml Wasser gelöstes Na₂CO₃, sowie 14,4 ml (238 mMol) Ethanolamin gegeben. Das sich alsbald bildende breiartige Reaktionsgemisch wird über Nacht bei Raumtemperatur gerührt und anderntags abgesaugt. Der Filterrückstand, der unumgesetztes Fmoc-O-Su, N-Hydroxysuccinimid sowie das gewünschte Produkt enthält, wird aus Essigester umkristallisiert. Man erhalt nach Trocknen im Vakuum 47,4 g = 76% der Theorie an reinem Produkt.
¹H-NMR (ppm) (DMSO):3.4 (m, CH₂O, 2 H); 3,6 (t, CH₂N, 2H); 4,2-4,5 (m, CH₂OCO + H [C9], 3 H); 5,2 (s [b], NH, 1 H); 7,2-7,9 (m, aromatisch, 8 H);

### Beispiel 2:

### Dichlor-N, N-diisopropylamino-phosphan

In einem 2 l-Dreihalsrundkolben mit 500ml Tropftrichter, KPG-Rührer, Thermometer und Aceton/Trockeneisbad werden 300 ml Äther, abs., 81 ml wasserfreies Pyridin und 87,5 ml PCl₃ (1 Mol) unter Rühren auf -70°C vorgekühlt. Man tropft dazu innerhalb von 2 Stunden 142 ml Diiospropylamin (1 Mol) in 250 ml abs. Äther und hält die Temperatur bei ca. -60 bis -65°C. Nach beendeter Zugabe läßt man das breiartig verdickte Reaktionsgemisch auf Raumtemperatur kommen und verdünnt zwecks besserer Rührbarkeit mit etwa 600 ml abs. Äther. Nach weiteren 3 Stunden Rühren bei Raumtemperatur wird der entstandene Niederschlag über eine Glasfritte abgesaugt, und mit Äther mehrfach gewaschen. Nach Abziehen des Äthers bei Normaldruck wird erst im Wasserstrahlvakuum von nicht umgesetztem PCl₃, Diisopropylamin und Pyridin befreit, sodann das verbleibende Öl im Ölpumpenvakuum fraktioniert destilliert. (Kₚ 46°C/0,35 Torr). Man erhält 73,4 g entsprechend 36% der Theorie des Phosphans.
31P-NMR (ppm) (CHCl₃):167,5

### Beispiel 3:

### 2-(9-Fluorenylmethoxycarbonyl-)aminoethyl-N, N-diisopropylamino-phosphochloridit

In einem 100 ml-Rundkolben werden 0,9 ml Dichlor-N, N-diisopropylamin-phosphan (5 mmol) in 30 ml abs. Tetrahydrofuran gelöst und dazu 0,4 ml wasserfreies Pyridin gegeben. Unter magnetischem Rühren tropft man zu diesem Gemisch bei Raumtemperatur eine Lösung von 1,4g 2-(9-Fluorenyl-methoxycarbonyl) aminoethanol (5 mmol) in 20 ml abs. Tetrahydrofuran langsam während ca. 5 Stunden zu. Nach Absaugen des abgeschiedenen Pyridinhydrochlorides und Abziehen des Tetrahydrofurans wird das verbleibende Öl (2,2g = 98% der Theorie) direkt zur Herstellung des Nucleosidphosphamidites eingesetzt (siehe Beispiel 4).

### Beispiel 4:

### 5′-O-Dimethoxytrityl-2′-desoxythymidin-3′-O-[2-(9-fluorenylmethoxycarbonyl)aminoethyl]-N, N-diisopropylaminophosphan

a) In einem 100 ml-Rundkolben werden 2,5 g 5′-O-Dimethoxytrityl-2′-desoxythymidin (4,6 mMol) in 50 ml Dichlormethan (über Na₂CO₃ destilliert) sowie 2,5 ml N-Ethyl-N, N-diisopropyl-amin gelöst. Dazu werden mit einer Einwegspritze 2 ml 2-(9-Fluorenylmethoxycarbonyl-) aminoethyl-N, N-diisopropylamino-phosphochloridit (ca. 5 mmol) gegeben. Man rührt 48 Stunden bei Raumtemperatur und dampft dann im Vakuum bis zum dickflüssigen Rückstand ein.
   Zu Aufreinigung des Rohproduktes wird an Kieselgel 60 chromatographiert (Säule 30 x 2 cm, Laufmittel Petroläther 50 - 75°C/Essigsäureethylester/Dichlormethan/Pyridin = 4 : 8 : 8 : 2. Die produktenthaltenden Fraktionen werden gesammelt, das Lösungsmittel restlos im Vakuum abgezogen.
   Man erhält 0,9 g entsprechend 20% der Theorie eines weißen, schaumigen Rückstandes.
b) In einem Alternativverfahren werden 5,45 g 5′-0-Dimethoxytrityl-2′-desoxythymidin (10 mmol) unter Rühren in 100 ml absolutem Dioxan gelöst. Dazu tropft man innerhalb von 30 Minuten eine Lösung von 2,7 g Bis (diisopropylamino)chlorphosphan (10 mmol), das nach S. Hammoto, H. Takaku, Chemistry Lett. 1986, 1401-1404 dargestellt wurde, und 2,1 ml Triethylamin (15 mmol) in 100 ml Dioxan. Die Umsetzung wird dünnschichtchromatographisch in Methylenchlorid/Essigsäureethylester = 1:1 als Laufmittel verfolgt. Nach 2 Stunden wird der Niederschlag aus Triethylammoniumchlorid unter Argonschutzgas abfiltriert und das Filtrat eingeengt (farbloser Schaum). Das gebildete 5′-0-Dimethoxytrityl-2′-desoxythymidin-3′-0-bis-(N,N-diisopropylamino)phosphan wird ohne weitere Isolierung zum gewünschten Produkt umgesetzt. Dazu wird der farblose Schaum in 100 ml absolutem Acetonitril aufgenommen und 3 g 2-(9-Fluorenylmethoxycarbonyl)-amino-ethanol (Beispiel 1) sowie 35 mg (5 mmol) Tetrazol (sublimiert) zugegeben. Man läßt über Nacht bei Raumtemperatur rühren und bricht dann die Reaktion durch Zugabe von 100 ml Essigsäureethylester ab. Nach dreimaliger Extraktion mit gesättigter Natriumchloridlösung werden die vereinigten organischen Phasen über Natriumsulfat getrocknet. Nach Abfiltrieren des Natriumsulfats wird das Filtrat eingeengt. Zur Reinigung des Rohprodukts wird über Kieselgel 60 H chromatographiert: (1 = 24 cm, d = 4 cm; Laufmittel: Methylenchlorid/Essigsäureethylester = 5:1). Nach Entfernen des Lösungsmittels im Vakuum erhält man wiederum einen farblosen Schaum. Dieser wird in 10 ml Methylenchlorid aufgenommen und mit 400 ml eiskaltem n-Hexan ausgefällt. Man erhalt 1.8 g entsprechend 20% der Theorie des gewünschten Produktes als farbloses Pulver.
   Die beiden Diastereomeren können sowohl im DC als auch im 31P-NMR unterschieden werden:
   Rf-Wert (CH₂Cl₂/EE = 1:1): 0,04, 0,15
   31P-NMR (ppm) (CD₃CN): 146,7, 145,8

### Beispiel 5:

### Synthese von d (Tp_{AE}TpTpTpTpTpTpTp_{AE}T)

Die Synthese des Oligonucleotids wurde im 1 uMol-Maßstab nach Standardprotokoll in einem vollautomatischen DNA-Synthesizer 8600 der Firma Biosearch durchgeführt. Hierzu wird prinzipiell das Synthese-Gerät mit einer mit 1 µmol Thymidin-Träger beschickten Reaktions-Säule bestückt und in einem ersten Reaktionsschritt die 5′-OH-Schutzgruppe (Dimethoxytrityl-) durch Behandlung mit einer 2%igen Dichloressigsäure-Lösung in Dichlormethan abgespalten. Nach Waschen der Säule mit Acetonitril erfolgt die Kupplung des im erfindungsgemäßen Sinne P-modifizierten 5′-O-dimethoxytriphenylmethyl-2′-desoxythymidin-3′-O-[2-(9-fluorenylmethoxy-carbonyl)aminoethyl]-N,N-diisopropylamino-phosphans aus Beispiel 4 unter gleichzeitiger Aktivierung mit Tetrazol in Acetonitril an die freie 5′-OH-Funktion des Start-Nucleosids. Das noch trivalent vorliegende P-Atom wird nach erneutem Waschen durch Oxidation mit einer Lösung von Jod in THF/Lutidin/H₂O in das natürliche pentavalente Phosphat überführt. Der nachfolgende Capping-Schritt mit Acetanhydrid/Dimethylaminopyridin blockiert durch Acetylierung nicht gekuppeltes 5′-OH-Nucleosid. Dadurch wird die Bildung von Fehlsequenzen unterdrückt. Nach Waschen beginnt mit erneuter Abspaltung der 5′-O-Dimethoxytrityl-Schutzgruppe der Synthesezyklus von vorne. In dieser Weise werden nun 6 Thymidin-Bausteine mit nicht-modifiziertem Phosphoamidit-Teil in die Reaktionsfolge eingebracht, bevor im letzten Zyklus eine weitere Kupplung mit dem aminoethylierten Thymidin-Phosphoamidit (Tp_{AE}) erfolgt. Nach beendeter Synthese wird durch Behandlung mit konzentrierter wässriger Ammoniaklösung das am Träger gebundene Oligonucleotid freigesetzt und gleichzeitig dabei auch die Fmoc-Schutzgruppe des aminoethylierten Phosphats entfernt. Es resultieren 86 ODE/A₂₆₀. Dieses Roh-Gemisch wurde unter folgenden Bedingungen mittels HPLC aufgearbeitet.
Säule: Mono Q HR 10/10 (Pharmacia)
Eluent A (Wasser), Eluent B (0,5 n-LiCl)
Gradient: von A in 60 Minuten auf 50% B.
Das Eluat wird über Nacht gegen H₂O dialysiert (Spektrapor, MWCO 1000)
Ausbeute: 55 ODE

### Beispiel 6:

### Markierung des Oligonucleotides aus Beispiel 5 mit Digoxigenin

55 ODE/A₂₆₀ des Oligomers aus Beispiel 5 werden in 1 ml 0,1 m-Na-boratpuffer pH 8,5 gelöst und mit einer Lösung von 10 mg Digoxigenin-O-succinyl-amidocapronsäure-N-hydroxysuccinimidester in 1 ml Dimethylformamid versetzt. Man rührt das Gemisch 18 Stunden bei Raumtemperatur, engt bis zur Trockene im Vakuum ein, löst in H₂O und trennt das Produktgemisch per HPLC:
Säule: Shandon Hypersil ODS, 25 cm x 0,4 cm
Eluent A: 0,1 m Triethylammoniumacetat-Lösung
Eluent B: 0,1 m Triethylammoniumacetat-Lösung/Isopropanol
Gradient: von A in 30 Minuten auf 50% B
Die Produktfraktion wird im Vakuum eingedampft, in Wasser aufgenommen und über Nacht gegen destilliertes Wasser dialysiert (Sprectrapor, MWCO 1000)
Ausbeute: 11 ODE/A₂₆₀

### Beispiel 7:

### Vergleich der Nachweisgrenze bei DNA-Nachweisen

Drei identische Oligonukleotide (38 mere) mit HIV spezifischer Sequenz wurden in ihren Hybridisierungseigenschaften gegen ein kloniertes HIV-DNA-Fragment (954bp PvuII/BglII-Fragment aus der gag-Region des HIV-Wfl.13-Isolats) getestet. An folgenden Stellen sind die Oligonukleotide mit Digoxigenin markiert:
1. Je an einem 5′-terminalen und einem in der Mitte lokalisierten Uracil (d.h. zwei Dig-Markierungen, Basenmarkierung an C-5 des Uracil).
2. Je an einem 5′-terminalen, 3′-terminalen und in der Mitte lokalisierten Uracil (3fache Dig-Markierung, Basenmarkierung an C-5 des Uracil).
3. Je an einer 5′-terminalen und in der Mitte lokalisierten Phosphatgruppe (2 Dig-Markierungen/Molekül, erfindungsgemäße Markierung).

### a) Hybridisierungsansatz für Oligonucleotide mit Dig-Markierung

Die Sample DNA wird entweder direkt auf Filter in Verdünnungsreihen von jeweils 1µl Volumen gespottet oder nach Auftrennung im Agaraose-Gel durch Southern Blot unter 20XSSC-Puffer auf die Filter transferiert. Die Fixierung erfolgt durch 3-minütige UV-Bestrahlung.

Die Filter werden unter folgenden Bedingungen prähybridisiert: 1 h bei 40°C in 5xSSC, 0,5% Blocking Reagens. Die anschließende Hybridisierung mit Digmarkierten Oligonucleotiden erfolgt unter folgenden Bedingungen: Über Nach bei 4°C in 5xSSC, 0,5% Blocking Reagens, 200 ng Oligonucleotide pro ml Hybridisierungslösung.

Die Filter werden danach 4x10 min in 2xSSC, 0,1% SDS bei 40°C gewaschen.

Die Detektion wird analog zu dem nicht-radioaktiven Markierungs- und Detektionskit (Boehringer Mannheim GmbH) mittels POD-markierten Antikörpers gegen Digoxigenin durchgeführt.

### b) Ergebnisse:

| Nachweisgrenze der gespotteten/geblotteten Sample DNA | |
|---|---|
| mit zweifach basenmarkiertem Oligonukleotid (1): | 10 ng |
| mit dreifach basenmarkiertem Oligonukleotid (2): | 10 ng |
| mit zweifach über Phosphat markiertem Oligonukleotid (3): | 1-10 ng |

## Patentansprüche

1. Verfahren zur Herstellung einer Nukleotidsequenz der Formel V 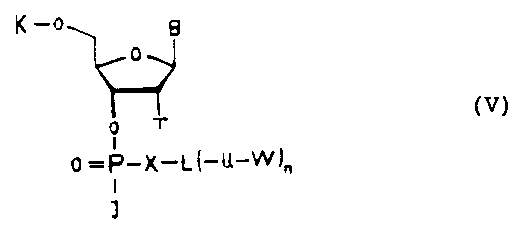 in der
K Wasserstoff oder das Phosphoratom des Phosphatrests eines weiteren Nukleotids oder einer Nukleotidsequenz,
J eine Hydroxygruppe oder ein 5'-Sauerstoffatom eines weiteren Nukleotids oder einer Nukleotidsequenz,
B eine natürliche oder modifizierte Nukleinbase,
T Wasserstoff, Niederalkyl, Azid, Niederalkoxy oder eine Hydroxylgruppe,
X Sauerstoff oder Schwefel,
L ein (n + 1)-valentes Brückenglied,
U Sauerstoff, Schwefel, Stickstoff oder N-H,
W ein detektierbarer Rest oder ein Rest ist, der in einen detektierbaren Rest überführt werden kann und
n eine natürliche Zahl von 1 bis 200 ist, wobei mehr als ein Nukleotidbaustein gemäß Formel V am Phosphoratom modifiziert ist,
durch Umsetzung einer Nukleotidsequenz der Formel IX, 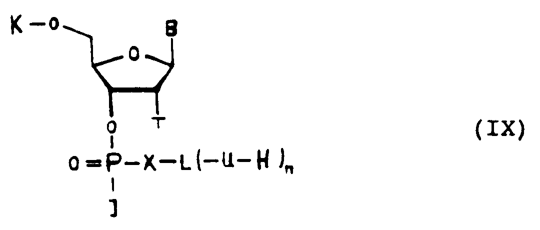 in der
K Wasserstoff oder das Phosphoratom des Phosphatrests eines weiteren Nukleotids oder einer Nukleotidsequenz,
J eine Hydroxygruppe oder ein 5'-Sauerstoffatom eines weiteren Nukleotids oder einer Nukleotidsequenz,
B eine natürliche oder modifizierte Nukleobase,
T Wasserstoff, Niederalkyl, Azid, Niederalkyloxy oder eine Hydroxygruppe,
X Sauerstoff oder Schwefel,
L ein (n + 1)-valentes Brückenglied,
U Sauerstoff, Schwefel, Stickstoff oder N-H und
n eine natürliche Zahl von 1 bis 200 bedeuten, wobei mehr als ein Nukleotidbaustein gemäß Formel IX am Phosphoratom modifiziert ist,
mit einer Verbindung der Formel IV
Y-W (IV),
wobei
Y eine reaktive Gruppe und
W ein detektierbarer Rest oder ein Rest ist, der in einen detektierbaren Rest überführt werden kann.

2. Nukleotidsequenz der Formel V 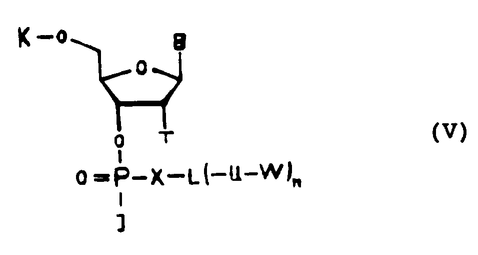 in der
K Wasserstoff oder das Phosphoratom des Phosphatrests eines weiteren Nukleotids oder einer Nukleotidsequenz,
J eine Hydroxygruppe oder ein 5'-Sauerstoffatom eines weiteren Nukleotids oder einer Nukleotidsequenz,
B eine natürliche oder modifizierte Nukleinbase,
T Wasserstoff, Niederalkyl, Azid, Niederalkoxy oder eine Hydroxylgruppe,
X Sauerstoff oder Schwefel,
L ein (n + 1)-valentes Brückenglied,
U Sauerstoff, Schwefel, Stickstoff oder N-H,
W ein detektierbarer Rest oder ein Rest ist, der in einen detektierbaren Rest überführt werden kann und
n eine natürliche Zahl von 1 bis 200 ist, wobei mehr als ein Nukleotidbaustein gemäß Formel V am Phosphoratom modifiziert ist.

3. Verfahren zu Herstellung von Nukleotidsequenzen der Formel IX 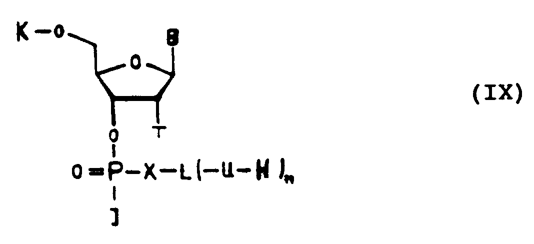 in der
K Wasserstoff oder das Phosphoratom des Phosphatrests eines weiteren Nukleotids oder einer Nukleotidsequenz,
J eine Hydroxygruppe oder ein 5'-Sauerstoffatom eines weiteren Nukleotids oder einer Nukleotidsequenz,
B eine natürliche oder modifizierte Nukleobase,
T Wasserstoff, Niederalkyl, Azid, Niederalkyloxy oder eine Hydroxygruppe,
X Sauerstoff oder Schwefel,
L ein (n + 1)-valentes Brückenglied,
U Sauerstoff, Schwefel, Stickstoff oder N-H und
n eine natürliche Zahl von 1 bis 200 bedeuten,
durch Reaktion einer Verbindung der Formel I 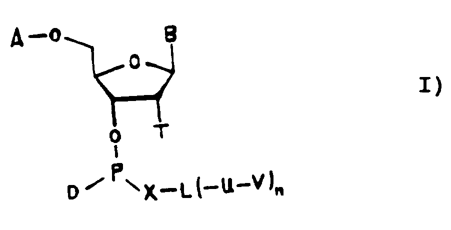 wobei
A eine Sauerstoffschutzgruppe, ein Nukleotid oder ein Oligonukleotid,
B eine natürliche oder modifizierte Nukleinbase,
X Sauerstoff oder Schwefel,
L ein (n + 1)-valentes Brückenglied,
T Wasserstoff oder eine gegebenenfalls geschützte Hydroxygruppe,
U Sauerstoff, Schwefel, Stickstoff oder N-H,
V eine abspaltbare Schutzgruppe,
n eine natürliche Zahl von 1 bis 200 und
D ein sekundärer Aminrest bedeuten,
mit einem weiteren Nukleosid, das eine freie 5'-Hydroxylgruppe aufweist und Oxidation der entstandenen Nukleotidsequenz.

4. Nukleotidsequenz der Formel IX 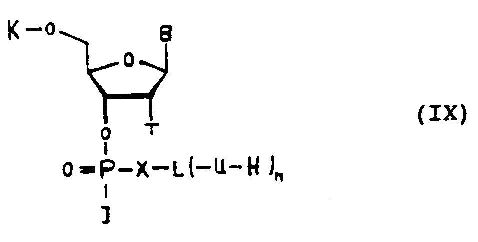 in der
K Wasserstoff oder das Phosphoratom des Phosphatrests eines weiteren Nukleotids oder einer Nukleotidsequenz,
J eine Hydroxygruppe oder ein 5'-Sauerstoffatom eines weiteren Nukleotids oder einer Nukleotidsequenz,
B eine natürliche oder modifizierte Nukleobase,
T Wasserstoff, Niederalkyl, Azid, Niederalkyloxy oder eine Hydroxygruppe,
X Sauerstoff oder Schwefel,
L ein (n + 1)-valentes Brückenglied,
U Sauerstoff, Schwefel, Stickstoff oder N-H und
n eine natürliche Zahl von 1 bis 200 bedeuten, wobei mehr als ein Nukleotidbaustein gemäß Formel IX am Phosphoratom modifiziert ist.

5. Nukleosidphosphoramidit der Formel I 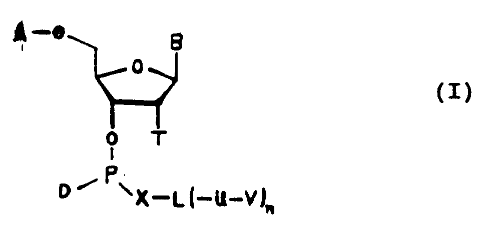 wobei
A eine Sauerstoffschutzgruppe, ein Nukleotid oder ein Oligonukleotid,
B eine natürliche oder modifizierte Nukleobase,
X Sauerstoff oder Schwefel,
L ein (n + 1)-valentes Brückenglied,
T Wasserstoff, Niederalkyl, Azid, Niederalkyloxy oder eine gegebenenfalls geschützte Hydroxygruppe,
U Sauerstoff, Schwefel, Stickstoff oder N-H,
V eine abspaltbare Schutzgruppe,
n eine natürliche Zahl von 1 bis 200 und
D ein sekundärer Aminrest bedeuten.

6. Verwendung von Nukleosidphosphoramiditen der Formel I zur Herstellung von Verbindungen der Formel V.

7. Verfahren zur Herstellung eines Nukleosidphosphoramidits der Formel I, durch Umsetzung einer Verbindung der Formel II 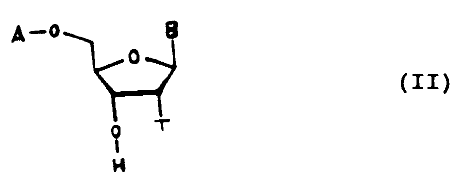 in der
A eine Sauerstoffschutzgruppe, ein Nukleotid oder ein Oligonukleotid,
B eine natürliche oder modifizierte Nukleinbase und
T Wasserstoff, Niederalkyl, Azid, Niederalkyloxy oder eine gegebenenfalls geschützte Hydroxygruppe,
bedeuten, mit einem Phosphan der Formel III 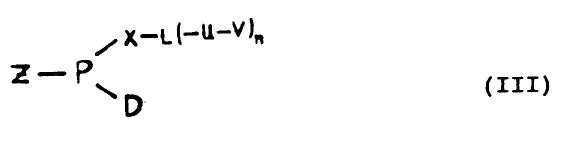 in der
Z eine gut austretende Gruppe,
X Sauerstoff oder Schwefel,
L ein mindestens bivalentes Brückenglied,
U Sauerstoff, Schwefel, Stickstoff oder N-H,
V eine abspaltbare Schutzgruppe,
n eine natürliche Zahl von 1 bis 200 und
D sekundärer Aminrest bedeuten.

8. Phosphan der Formel III 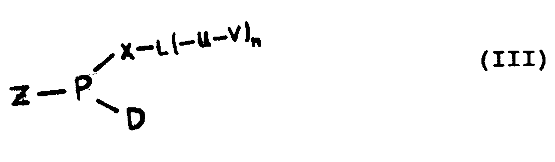 in der
Z Halogen,
X Sauerstoff oder Schwefel,
L ein mindestens bivalentes Brückenglied,
U Sauerstoff, Schwefel, Stickstoff oder N-H,
V eine abspaltbare Schutzgruppe,
n eine natürliche Zahl von 1 bis 200 und
D ein sekundärer Aminrest bedeuten.

9. Verfahren zur Herstellung von Phosphanen, dadurch gekennzeichnet, daß eine Verbindung der Formel (VI)
P(-Z)₃ (VI)
in der Z eine gut austretende Gruppe bedeutet, mit einem sekundären Amin der Formel (VII)
H-D (VII)
in der
D ein sekundärer Aminrest bedeuten,
umsetzt, und das Produkt mit einer Verbindung der Formel VIII
H-X-L(-U-V)ₙ (VIII)
in der
X Sauerstoff oder Schwefel,
L ein (n + 1)-valentes Brückenglied,
U Sauerstoff, Schwefel, Stickstoff oder N-H,
V eine abspaltbare Schutzgruppe,
n eine natürliche Zahl von 1 bis 200
bedeuten, reagieren läßt und das entstandene Produkt abtrennt.

10. Verwendung einer Nukleotidsequenz der Formel (V) 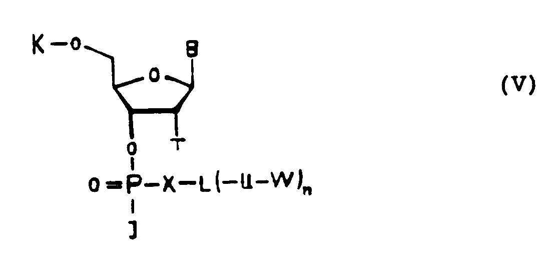 in der
K Wasserstoff oder das Phosphoratom des Phosphatrests eines weiteren Nukleotids oder einer Nukleotidsequenz,
J eine Hydroxygruppe oder ein 5'-Sauerstoffatom eines weiteren Nukleotids oder einer Nukletoidsequenz,
B eine natürliche oder modifizierte Nukleinbase,
T Wasserstoff, Niederalkyl, Azid, Niederalkoxy oder eine Hydroxylgruppe bedeuten und
X, L, U, W und n die im Anspruch 2 angegebene Bedeutung haben, wobei mehr als ein Nukleotidbaustein gemäß Formel V am Phosphoratom modifiziert ist,
zum Nachweis einer zu dieser Nukleotidsequenz im wesentlichen komplementären Nukleotidsequenz.

11. Reagenz zum Nachweis einer Nukleinsäure in einer Probe durch Inkontaktbringen der Probe mit einer dazu im wesentlichen komplementären Nukleinsäure, dadurch gekennzeichnet, daß es als zur Proben-DNA komplementäre Nukleinsäure eine Nukleotidsequenz gemäß Anspruch 2 enthält.

12. Verwendung einer Nukleotidsequenz der Formel (V) 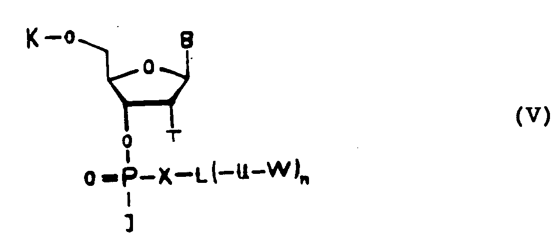 wobei diese Sequenz wie im Anspruch 2 definiert ist, als Primer in der enzymatischen Synthese von doppelsträngigen Nukleinsäuren.

## Claims

1. Process for the preparation of a nucleotide sequence of the formula V 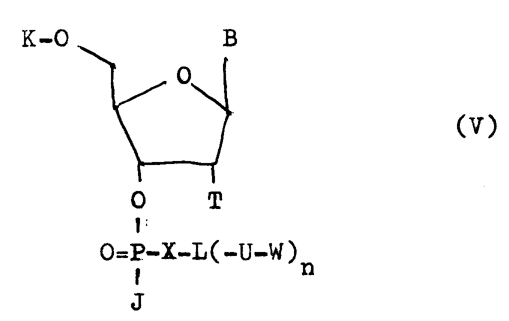 in which K is hydrogen or the phosphorus atom of the phosphate residue of a further nucleotide or of a nucleotide sequence, J a hydroxyl group or a 5'-oxygen atom of a further nucleotide or of a nucleotide sequence, B a natural or modified nucleic base, T hydrogen, lower alkyl, azide, lower alkoxy or a hydroxyl group, X oxygen or sulphur, L an (n + 1)-valent bridge member, U oxygen, sulphur, nitrogen or N-H, W a detectable residue or a residue which can be converted into a detectable residue and n a natural number of 1 to 200, whereby more than one nucleotide component is modified on the phosphorus atom according to formula V, by reaction of a nucleotide sequence of the formula IX, 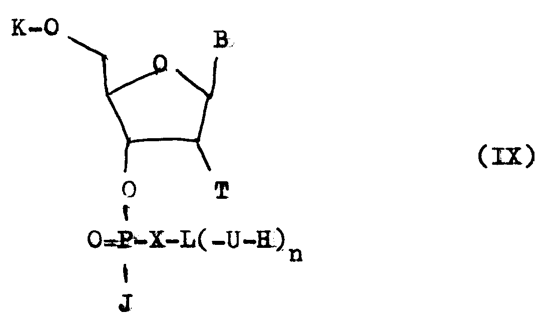 in which K is hydrogen or the phosphorus atom of the phosphate residue of a further nucleotide or of a nucleotide sequence, J a hydroxyl group or a 5'-oxygen atom of a further nucleotide or of a nucleotide sequence, B a natural or modified nucleobase, T hydrogen, lower alkyl, azide, lower alkyloxy or a hydroxyl group, X oxygen or sulphur, L an (n + 1)-valent bridge member, U oxygen, sulphur, nitrogen or N-H and n a natural number of 1 to 200, whereby more than one nucleotide component is modified on the phosphorus atom, according to formula IX with a compound of the formula IV
Y-W (IV),
whereby Y is a reactive group and W is a detectable residue or a residue which can be converted into a detectable residue.

2. Nucleotide sequence of the formula V 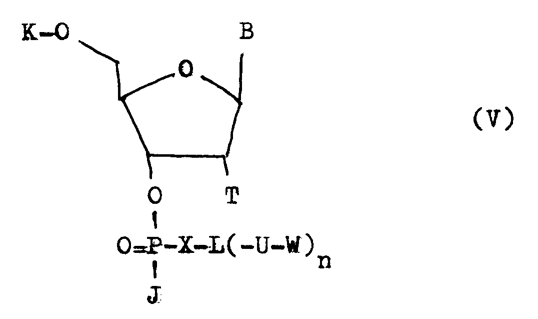 in which K is hydrogen or the phosphorus atom of the phosphate residue of a further nucleotide or of a nucleotide sequence, J a hydroxyl group or a 5'-oxygen atom of a further nucleotide or of a nucleotide sequence, B a natural or modified nucleic base T hydrogen, lower alkyl, azide, lower alkoxy or a hydroxyl group, X oxygen or sulphur, L an (n + 1)-valent bridge member, U oxygen, sulphur, nitrogen or N-H, W a detectable residue or a residue which can be converted into a detectable residue and n a natural number of 1 to 200, whereby more than one nucleotide component is modified on the phosphorus atom according to formula V.

3. Process for the preparation of nucleotide sequences of the formula IX 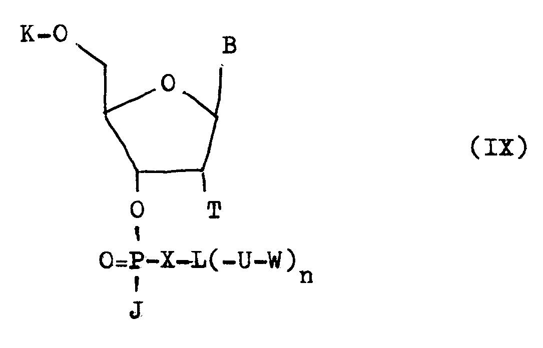 in which K signifies hydrogen or the phosphorus atom of the phosphate residue of a further nucleotide or of a nucleotide sequence, J a hydroxyl group or a 5'-oxygen atom of a further nucleotide or of a nucleotide sequence, B a natural or modified nucleobase, T hydrogen, lower alkyl, azide, lower alkyloxy or a hydroxyl group, X oxygen or sulphur, L an (n + 1)-valent bridge member, U oxygen, sulphur, nitrogen or N-H and n a natural number from 1 to 200, by reaction of a compound of the formula I 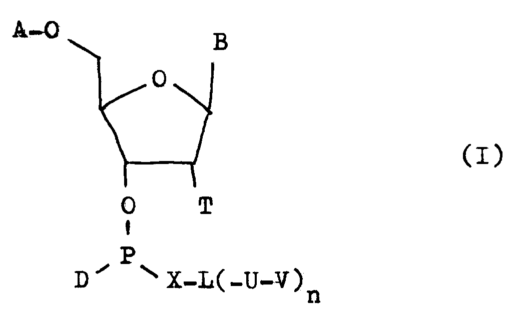 whereby A signifies an oxygen protective group, a nucleotide or an oligonucleotide, B a natural or modified nucleic base, X oxygen or sulphur, L an (n + 1)-valent bridge member, T hydrogen or a possibly protected hydroxyl group, U oxygen, sulphur, nitrogen or N-H, V a protective group which can be split off, n a natural number from 1 to 200 and D a secondary amine residue, with a further nucleoside which has a free 5'-hydroxyl group and oxidation of the resulting nucleotide sequence.

4. Nucleotide sequence of the formula IX 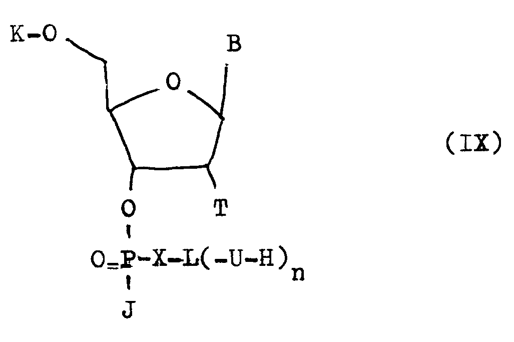 in which K signifies hydrogen or the phosphorus atom of the phosphate residue of a further nucleotide or of a nucleotide sequence, J a hydroxyl group or a 5'-oxygen atom of a further nucleotide or of a nucleotide sequence, B a natural or modified nucleobase, T hydrogen, lower alkyl, azide, lower alkyloxy or a hydroxyl group, X oxygen or sulphur, L an (n + 1)-valent bridge member, U oxygen, sulphur, nitrogen or N-H and n a natural number from 1 to 200, whereby more than one nucleotide component is modified on the phosphorus atom according to formula IX.

5. Nucleoside phosphoramidite of the formula I 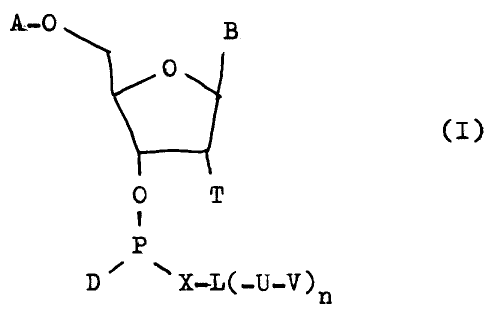 whereby A signifies an oxygen protective group, a nucleotide or an oligonucleotide, B a natural or modified nucleobase, X oxygen or sulphur, L an (n + 1)-valent bridge member, T hydrogen, lower alkyl, azide, lower alkyloxy or a possibly protected hydroxyl group, U oxygen, sulphur, nitrogen or N-H, V a protective group which can split off, n a natural number from 1 to 200 and D a secondary amine residue.

6. Use of nucleoside phosphoramidites of the formula I for the preparation of compounds of the formula V.

7. Process for the preparation of a nucleoside phosphoramidite of the formula I by reaction of a compound of the formula II 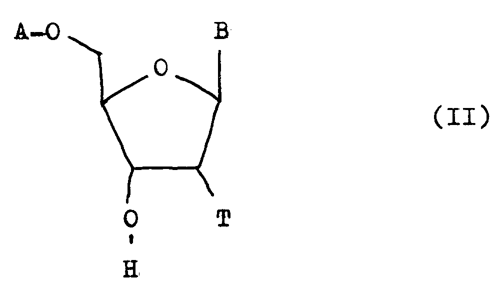 in which A signifies an oxygen protective group, a nucleotide or an oligonucleotide, B a natural or modified nucleic base and T hydrogen, lower alkyl, azide, lower alkyloxy or a possibly protected hydroxyl group, with a phosphane of the formula III 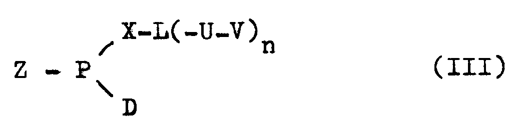 in which Z signifies a readily removable group, X oxygen or sulphur, L an at least bivalent bridge member, U oxygen, sulphur, nitrogen or N-H, V a protective group which can be split off, n a natural number from 1 to 200 and D secondary amine residue.

8. Phosphane of the formula III 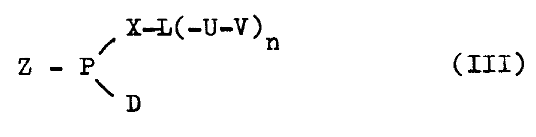 in which Z signifies halogen, X oxygen or sulphur, L an at least bivalent bridge member, U oxygen, sulphur, nitrogen or N-H, V a protective group which can be split off, n a natural number from 1 to 200 and D a secondary amine residue.

9. Process for the preparation of phosphanes, characterised in that a compound of the formula (VI)
P(-Z)₃ (VI)
in which Z signifies a readily removable group, is reacted with a secondary amine of the formula (VII)
H-D (VII)
in which D signifies a secondary amine residue, and the product is allowed to react with a compound of the formula VIII
H-X-L(-U-V)ₙ (VIII)
in which X signifies oxygen or sulphur, L an (n + 1)-valent bridge member, U oxygen, sulphur, nitrogen or N-H, V a protective group which can be split off, n a natural number from 1 to 200, and the resulting product separated off.

10. Use of a nucleotide sequence of the formula (V) 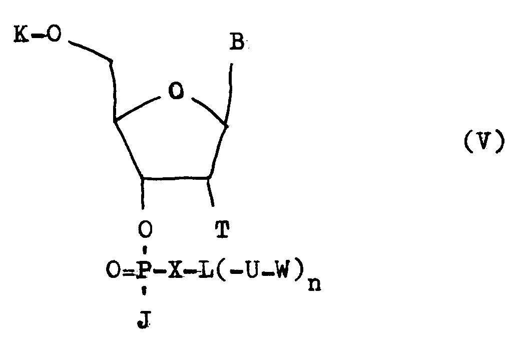 in which K signifies hydrogen or the phosphorus atom of the phosphate residue of a further nucleotide or of a nucleotide sequence, J a hydroxyl group or a 5'-oxygen atom of a further nucleotide or nucleotide sequence, B a natural or modified nucleic base, T hydrogen, lower alkyl, azide, lower alkoxy or a hydroxyl group and X, L, U, W and n have the meaning given in claim 2, whereby more than one nucleotide component according to formula V is modified on the phosphorus atom, for the detection of a nucleotide sequence substantially complementary to this nucleotide sequence.

11. Reagent for the detection of a nucleic acid in a sample by bringing into contact of the sample with a nucleic acid substantially complementary thereto, characterised in that, as nucleic acid complementary to the sample DNA, it contains a nucleotide sequence according to claim 2.

12. Use of a nucleotide sequence of the formula (V) 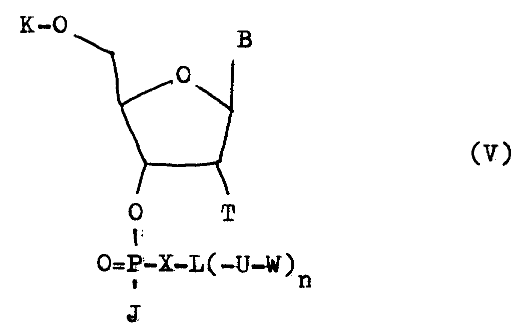 whereby this sequence is as defined in claim 2, as primer in the enzymatic synthesis of double-stranded nucleic acids.

## Revendications

1. Procédé de préparation d'une séquence de nucléotide de formule V 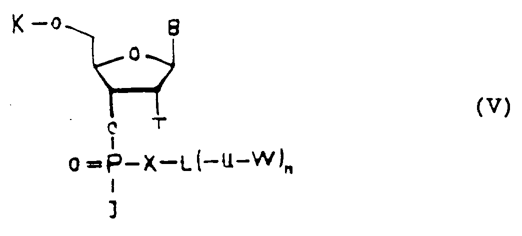 dans laquelle
K représente un atome d'hydrogène ou l'atome de phosphore du reste phosphate d'un autre nucléotide ou d'une séquence de nucléotide,
J représente un groupe hydroxy ou un atome d'oxygène en 5' d'un autre nucléotide ou d'une séquence de nucléotide,
B une base de nucléotide naturelle ou modifiée,
T un atome d'hydrogène, un groupe alkyle inférieur, azido, alcoxy inférieur ou hydroxyle,
X représente un atome d'oxygène ou de soufre,
L représente un élément de pont possédant (n+1) valences,
U représente un atome d'oxygène, de soufre, d'azote ou un groupe N-H,
W représente un reste détectable ou un reste qui peut être transformé en un reste détectable,
n représente un nombre naturel de 1 à 200, un nombre supérieur à un de motif de nucléotide de formule V étant modifié sur l'atome de phosphore,
consistant en ce que l'on fait réagir une séquence de nucléotide de formule IX 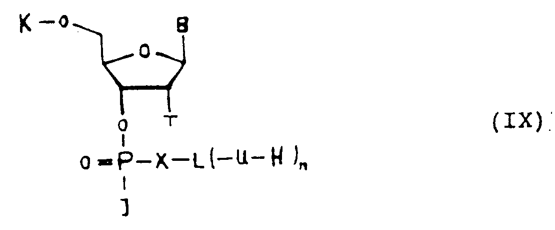 dans laquelle
K représente un atome d'hydrogène ou l'atome de phosphore du reste phosphate d'un autre nucléotide ou d'une séquence de nucléotide,
J représente un groupe hydroxy ou un atome d'oxygène en 5' d'un autre nucléotide ou d'une séquence de nucléotide,
B une base de nucléotide naturelle ou modifiée,
T un atome d'hydrogène, un groupe alkyle inférieur, azido, alcoxy inférieur ou hydroxyle,
X représente un atome d'oxygène ou de soufre,
L représente un élément de pont possédant (n+1) valences,
U représente un atome d'oxygène, de soufre, d'azote ou un groupe N-H,
n représente un nombre naturel de 1 à 200, un nombre supérieur à un de motif de nucléotide de formule V étant modifié sur l'atome de phosphore,
avec un composé de formule IV
Y-W (IV),
dans laquelle
Y représente un groupe réactif, et
W représente un reste détectable ou un reste qui peut être transformé en un reste détectable.

2. Nucléotide de formule V 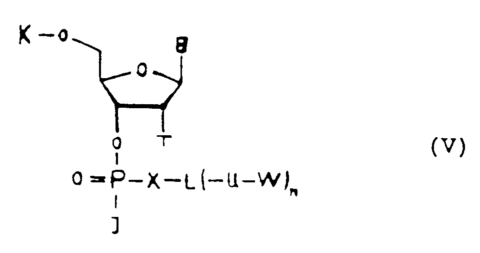 dans laquelle
K représente un atome d'hydrogène ou l'atome de phosphore du reste phosphate d'un autre nucléotide ou d'une séquence de nucléotide,
J représente un groupe hydroxy ou un atome d'oxygène en 5' d'un autre nucléotide ou d'une séquence de nucléotide,
B une base de nucléotide naturelle ou modifiée,
T un atome d'hydrogène, un groupe alkyle inférieur, azido, alcoxy inférieur ou hydroxyle,
X représente un atome d'oxygène ou de soufre,
L représente un élément de pont possédant (n+1) valences,
U représente un atome d'oxygène, de soufre, d'azote ou un groupe N-H,
W représente un reste détectable ou un reste qui peut être transformé en un reste détectable,
n représente un nombre naturel de 1 à 200, un nombre supérieur à un de motif de nucléotide de formule V étant modifié sur l'atome de phosphore.

3. Procédé de préparation de séquences de nucléotide de formule IX 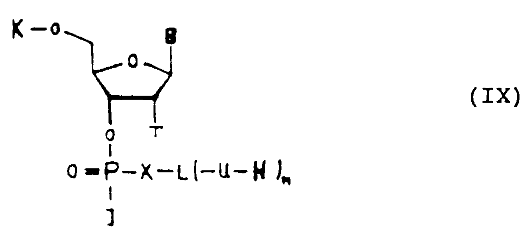 dans laquelle
K représente un atome d'hydrogène ou l'atome de phosphore du reste phosphate d'un autre nucléotide ou d'une séquence de nucléotide,
J représente un groupe hydroxy ou un atome d'oxygène en 5' d'un autre nucléotide ou d'une séquence de nucléotide,
B une base de nucléotide naturelle ou modifiée,
T un atome d'hydrogène, un groupe alkyle inférieur, azido, alcoxy inférieur ou hydroxyle,
X représente un atome d'oxygène ou de soufre,
L représente un élément de pont possédant (n+1) valences,
U représente un atome d'oxygène, de soufre, d'azote ou un groupe N-H,
W représente un reste détectable ou un reste qui peut être transformé en un reste détectable,
n représente un nombre naturel de 1 à 200, un nombre supérieur à un de motif de nucléotide de formule V étant modifié sur l'atome de phosphore,
consistant à faire réagir un composé de formule I 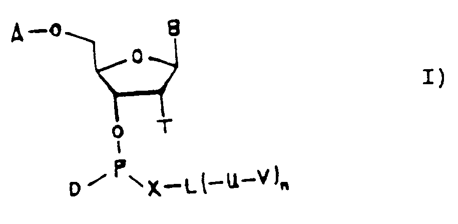 dans laquelle
A représente un groupe protecteur d'oxygène, un nucléotide ou un oligonucléotide,
B représente une base de nucléotide naturelle ou modifiée,
X représente un atome d'oxygène ou de soufre,
L représente un élément de pont possédant (n+1) valences,
T représente un atome d'hydrogène ou un groupe hydroxy éventuellement protégé,
U représente un atome d'oxygène, de soufre, d'azote ou N-H,
V représente un groupe de protection éliminable,
n représente un nombre naturel de 1 à 200, et
D représente un reste d'amine secondaire,
avec un autre nucléoside, qui présente un groupe 5'-hydroxyle libre,
et à oxyder la séquence de nucléotide formée.

4. Séquence de nucléotide de formule IX 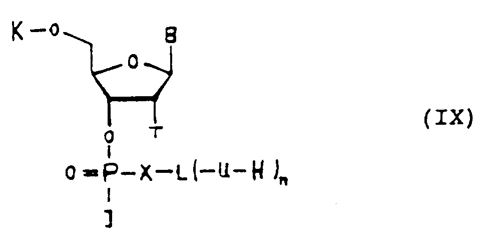 dans laquelle
K représente un atome d'hydrogène ou l'atome de phosphore du reste phosphate d'un autre nucléotide ou d'une séquence de nucléotide,
J représente un groupe hydroxy ou un atome d'oxygène en 5' d'un autre nucléotide ou d'une séquence de nucléotide,
B une base de nucléotide naturelle ou modifiée,
T un atome d'hydrogène, un groupe alkyle inférieur, azido, alcoxy inférieur ou hydroxyle,
X représente un atome d'oxygène ou de soufre,
L représente un élément de pont possédant (n+1) valences,
U représente un atome d'oxygène, de soufre, d'azote ou un groupe N-H,
n représente un nombre naturel de 1 à 200, un nombre supérieur à un de motif de nucléotide de formule V étant modifié sur l'atome de phosphore.

5. Nucléoside phosphoramidite de formule I 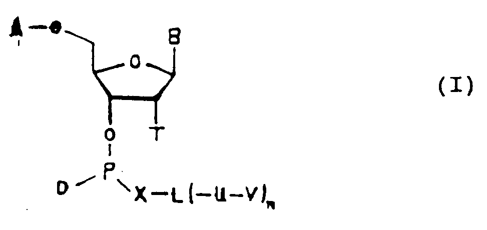 dans laquelle
A représente un groupe protecteur d'oxygène, un nucléotide ou un oligonucléotide,
B représente une base de nucléotide naturelle ou modifiée,
X représente un atome d'oxygène ou de soufre,
L représente un élément de pont possédant (n+1) valences,
T représente un atome d'hydrogène ou un groupe hydroxy éventuellement protégé,
U représente un atome d'oxygène, de soufre, d'azote ou N-H,
V représente un groupe de protection éliminable,
n représente un nombre naturel de 1 à 200, et
D représente un reste d'amine secondaire.

6. Utilisation de nucléoside phosphoramidites de formule I pour la préparation de composés de formule V.

7. Procédé de préparation de nucléoside phosphoramidites de formule I, consistant à faire réagir un composé de formule II 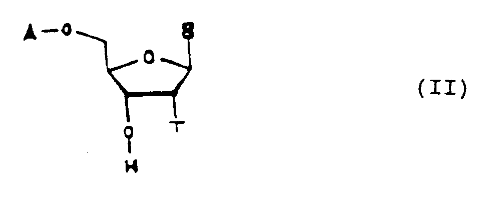 dans laquelle
A représente un groupe protecteur d'oxygène, un nucléotide ou un oligonucléotide,
B représente une base de nucléotide naturelle ou modifiée, et
T un atome d'hydrogène, un groupe alkyle inférieur, azido, alcoxy inférieur ou un groupe hydroxyle éventuellement protégé,
avec un phosphane de formule III 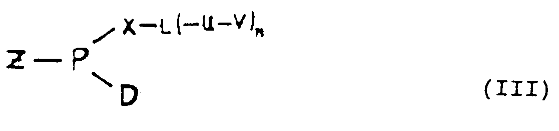 dans laquelle
Z représente un groupe s'éliminant facilement,
X représente un atome d'oxygène ou de soufre,
L représente un élément de pont au moins bivalent,
U représente un atome d'oxygène, de soufre, d'azote ou N-H,
V représente un groupe protecteur éliminable,
n représente un nombre naturel de 1 à 200, et
D représente un reste d'amine secondaire.

8. Phosphane de formule III 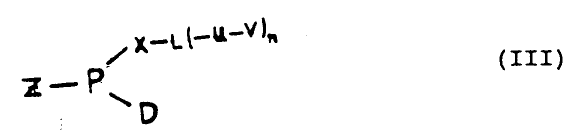 dans laquelle
Z représente un atome d'halogène,
X représente un atome d'oxygène ou de soufre,
L représente un élément de pont au moins bivalent,
U représente un atome d'oxygène, de soufre, d'azote ou N-H,
V représente un groupe protecteur éliminable,
n représente un nombre naturel de 1 à 200, et
D représente un reste d'amine secondaire.

9. Procédé de préparation de phosphanes, caractérisé en ce qu'on fait réagir un composé de formule (VI)
P(-Z)₃ (VI)
dans laquelle Z représente un groupe éliminable facilement, avec une amine secondaire de formule (VII)
H-D (VII)
dans laquelle
D représente une amine secondaire,
et on fait réagir le produit formé avec un composé de formule VIII
H-X-L(-U-V)ₙ (VIII)
dans laquelle
X représente un atome d'oxygène ou de soufre,
L représente un élément de pont possédant (n+1) valences,
U représente un atome d'oxygène, de soufre, d'azote ou un groupe N-H,
V représente un groupe protecteur éliminable,
n représente un nombre naturel de 1 à 200,
et on sépare le produit obtenu.

10. Utilisation d'une séquence de nucléotide de formule (V) 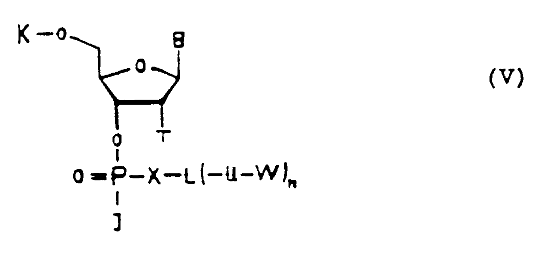 dans laquelle
K représente un atome d'hydrogène ou l'atome de phosphore du reste phosphate d'un autre nucléotide ou d'une séquence de nucléotide,
J représente un groupe hydroxy ou un atome d'oxygène en 5' d'un autre nucléotide ou d'une séquence de nucléotide,
B une base de nucléotide naturelle ou modifiée,
T un atome d'hydrogène, un groupe alkyle inférieur, azido, alcoxy inférieur ou hydroxyle,
X, L, U, W et n ayant les significations indiquées dans la revendication 2,
un nombre supérieur à un de motif de nucléotide de formule V étant modifié sur l'atome de phosphore.

11. Réactif pour la détermination d'un acide nucléique dans une sonde, par mise en contact de la sonde avec un acide nucléique essentiellement complémentaire de celle-ci, caractérisé en ce que, comme acide nucléique complémentaire de la sonde ADN, il contient une séquence de nucléotide selon la revendication 2.

12. Utilisation d'une séquence de nucléotide de formule (V) 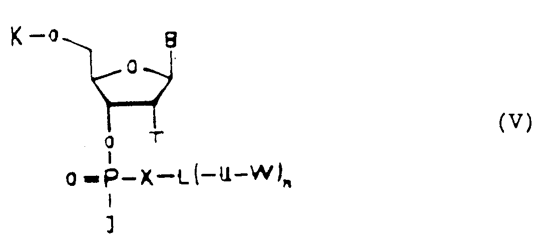 dans laquelle cette séquence est définie comme dans la revendication 2, en tant qu'amorce dans la synthèse enzymatique d'acides nucléiques double brin.
